# EUROPEAN PATENT SPECIFICATION

(11) **EP 0 496 796 B1**
(45) Date of publication and mention of the grant of the patent: **31.08.1994**
(21) Application number: 90915877.6
(22) Date of filing: 17.10.1990
(51) Int. Cl.: A61K 31/135, A61K 31/40, A61K 31/44

(54) **METHOD AND COMPOSITIONS FOR INHIBITION OF DISORDERS ASSOCIATED WITH OXIDATIVE DAMAGE**
VERFAHREN UND ZUBEREITUNGEN ZUR HEMMUNG VON MIT OXIDATIVER SCHÄDIGUNG ASSOZIIERTEN KRANKHEITEN
PROCEDE ET COMPOSITIONS INHIBANT LES TROUBLES ASSOCIES AUX LESIONS OXYDATIVES DES TISSUS

(30) Priority: 17.10.1989 US 422651; 27.09.1990 US 589177
(43) Date of publication of application: 05.08.1992
(73) Proprietor: Oklahoma Medical Research Foundation, Oklahoma City Oklahoma 73104 (US); UNIVERSITY OF KENTUCKY RESEARCH FOUNDATION, Lexington, Kentucky 40506-0032 (US)
(72) Inventor: CARNEY, John, M., Lexington, KY 50403 (US); FLOYD, Robert, A., Oklahoma City, OK 73120 (US)
(74) Representative: Bassett, Richard Simon
(86) International application number: PCT/US90/05952
(87) International publication number: WO 91/05552

(56) References cited:
- WO-A-88/05044
- E.K.Lai et al, Archives of Biochemistry and Biophysics, Vol. 244, N 1, January, pp. 156-160, 1986
- J.Clin.Invest. vol. 82, no. 2, August 1988, The American Soc. for Clinical Investigations, Inc. R. Bolli et al., pages 476485; page 477, page 484
- Chemical Abstracts, vol. 107, no. 7, 17. Aug. 1987, C.Chiu et al., Transplant. Proc. 1987, 19
- Dialog Inform. Services, File 155: Medline, AN no. 05997907 K.McKechnie et al.
- Dialog Inform. Services, Inc., File 155, Medline An no. 06790055, G.P.Novelli et al. see the abtract
- Chemical Abstracts, vol. 107, no. 7, 17. August 1987, D.J. Hearse et al., see page 49, abstract 51716y
- Circulatory Shock, vol. 29, no. 4, December 1989, Alan R.Liss, Inc., S.A. Hamburger et al., pages 329-334, see the whole article
- Chemical Abstracts, vol. 101, no. 5. 30. July 1984, R.Sridhar et al.see page 180, abstract 3459r

## Description

The present invention is a method and compositions containing spin trapping agents for the treatment of central nervous system tissue damage.

Oxygenated tissue suffers damage, in many cases permanent damage, if it becomes ischemic and is then reperfused. Oxygen free radicals have been implicated in the injury. Brain appears to be uniquely susceptible to ischemia/reperfusion injury, and neurons are more susceptible than glial cells. Certain areas of the brain, for example, the hippocampus and spinal cord, are more susceptible than other regions of the brain. As a result, ischemia/reperfusion injury to brain may have a multiplicative effect simply because of the necessity for complete integrity of all regions in order to have proper functioning.

Several mechanisms may cause ischemic brain damage. For example, the mechanisms responsible for selective neuronal vulnerability may be different from those that cause glial swelling and extensive brain edema, and a third set of events may underlie such gross functional aberrations as seizures. Siesjo, Critical Care Med. 16, 954-963 (1988), has grouped the numerous observations relating to brain injury into four possible mechanisms. These include: A) calcium mediated cell death, B) excitotoxic damage (glutamate buildup), C) free radical events and D) acidosis. It is likely that all four basic mechanisms are either interrelated or contribute collectively to the injury observed. With regard to free radical mechanisms in brain injury, Siesjo concluded that evidence supporting the involvement of free radical mechanisms was not definitive.

Free radicals have been postulated to be mediators of reperfusion damage. The important production sites of such radicals as the superoxide (0⁻²) and hydroxyl (OH⁻) species are the mitochondrial respiratory chain and the sequences catalyzed by cyclooxygenase and lipoxygenase. However, radicals are also formed during autoxidation of many compounds (e.g., catecholamines). Several ischemic events favor a spurt of free-radical formation, e.g., those causing oxidation of polyenoic free fatty acids, release and reuptake of catecholamines, and oxidation of hypoxanthine by xanthine oxidase. Although all these events occur during recirculation, when the O₂ supply is restored, they represent metabolic cascades triggered by agonist-receptor interactions, energy failure, and/or calcium influx during the insult. Although free radical formation is a likely cause of ischemic damage, it has been difficult to directly demonstrate that such formation occurs and/or that it is sufficiently pronounced to overwhelm the antioxidative defense of the tissue, Curran, et al., Mol. Cell. Biol. 5, 167-172 (1985). In recent years, however, evidence has been obtained that ischemia may cause conjugated dienes and malondialdehyde to accumulate in the tissue. Nonetheless, it remains to be conclusively shown that free-radical damage to unsaturated acyl chains in phospholipids, to protein, or to nucleic acids constitutes an important part of the ischemic necrosis. At present, the evidence is relatively strong for an involvement of free-radical mechanisms in vascular injury, and less so in damage affecting nerve and glial cells.

Reviews by Hall and Braughler, Free Radical Biol. Med. 6, 303-313 (1989), Kontos, Physiology of Oxygen Radicals, ed. Taylor, Matalon and Ward, pp. 207-216 (Am. Physiol. Soc. Bethesda, MD 1986), and Ernster, Critical Care Med. 16, 947-953 (1988), document a significant amount of evidence implicating oxidative damage in head and spinal cord injury, as well as in hemorrhagic stroke and ischemic stroke. Most research which strongly implicates the primary role of oxidative damage in ischemia/reperfusion injury in brain can be grouped into either of two types of studies: those which show protection by addition of agents preventing peroxidative events, or those designed to observe free radicals.

Although no drugs are currently approved for clinical use in treating tissue damage due to ischemia, several compounds have been proposed as potentially being effective. Mannitol, an oxygen scavenger, has been added to reperfusion media to limit damage to organs for transplantation. Superoxide dismutase (SOD) has been suggested as a means for limiting in vivo oxidative damage. The most promising compounds that interfere with peroxidation generation are the lazaroides, modified prednisones, described by J. M. McCall, Acta Anesthesia Belgica, First Antwerp Int. Trauma Symp., which have been reported to be efficacious if given during or after ischemia. For example, Hall, et al, Stroke 19, 997-1002 (1988), demonstrated that the 21-aminosteroid (474006F), which is known to prevent peroxidation in brain homogenate, as described by Braughler, et al., J. Biol. Chem. 262, 10438-10440 (1987), protected gerbils against brain damage induced by three hours of unilateral carotid artery occlusion. White and Aust and co-workers, Adv. Free Radical Biol. Med. 1,1-17 (1985), and Babbs, Resuscitation 13, 165-173 (1986), have demonstrated that iron chelators protect animals from ischemia/reperfusion injury.

With regard to direct demonstrations of oxidative events during ischemia/reperfusion injury in brain, there are pertinent observations using spin-trapping techniques, salicylate hydroxylation, protein oxidation, and nucleic acid oxidative damage. Spin-trapping has provided clear evidence implicating free radical production during ischemia/reperfusion injury. Imaizumi, et al, Neurological Res. 8, 214-220 (1986), showed that the spin-trap PBN when incubated with rat brain homogenate of animals which had experienced very low oxygen pressure for 5 min was able to trap an apparently lipid-type radical. Kirsch, et al, Pediatric Res. 21, 202A (1987), stated in a preliminary note that PBN had trapped free radicals in post ischemic brains from animals pretreated with the spin-trap. McCay's group have spin trapped free radicals in mouse brain, Arch. Biochem. Biophys. 244, 156-160 (1986), and shown that spin-trapped free radicals of PBN are present in blood leaving the heart of intact dogs recovering from a "stunned" myocardium , J. Clin. Invest. 82, 476-485 (1988). As described, the free radicals spin-trapped are apparently lipid-type free radicals and appear within one minute after release of occlusion and reach peak intensity about 5 minutes after reperfusion starts. PBN enhanced recovery of the post ischemic function of the "stunned" heart.

The effect of agents on hepatic ischemia was studied by Chiu *et al*, Chemical Abstracts, 107:57625t, 1987. Administration of PBN immediately before the onset of ischemia was found to improve hepatic performance following 40 minutes ischemia in dogs.

McKechnie *et al*, Dialog Information Services, File 155: MEDLINE, 05997907 and Circ. Shock 1986, 19(4), p 429-439 investigating the possible role of oxygen free radicals in endotoxemia found that PBN improved survival of rats following endotoxin shock. Novelli *et al*, Dialog Information Services, File 155: MEDLINE, 06790055 and Free Rad, Res. Commun, 1986, 1(5), p 321-327, found that PBN was effective in traumatic shock. WO-A-88/05044 disclosed the use of stable nitroxides for breaking and preventing ischemic cell damage in a reperfusion situation.

Hearse *et al*, Chemical Abstracts, 107: 57716y, 1987 found that the spin-trap DMPO reduced reperfusion-induced arrythmias following ischemia in isolated rat heart. Lai et al, Archives of Biochemistry and Biophysics, Vol. 244, N^{°} 1, pp. 156-160 (1986) have studied in vivo spin trapping of free radicals generated in brain during γ radiation of mice, using PBN.

While PBN has been used in a number of research studies, there has been no data to support the proposition that it, or any other spintrapping agents, could be useful *in vivo*, with respect to treatment of tissue damage in the central nervous system. *In vivo*, the drug must be able to (1) cross the blood brain barrier and (2) act in a manner which reduces tissue damage during or following ischemia.

Age related changes in central nervous system function have generally been associated with the loss of cells, a widening of lateral ventricles and deficits in short term memory. The precise mechanisms of functional changes as a result of aging, or other diseases associated with aging, have not generally been agreed upon. Several mechanisms for the generation of oxidized material in the brain have been proposed. In particular, transition metals, especially iron and copper, have been suggested as mediating aspects of this oxidation. A marked reduction in certain neurotransmitter receptor systems has been associated with increased oxidation of proteins. For example, decreases in muscarinic receptors and other cholinergic systems have been characterized as they relate to alterations in functions in Alzheimers disease. It has also been hypothesized that aging is associated with multiple minor periods of ischemia (multi-infarct conditions or transient ischemia attacks) which, over a period of time, may give rise to the production of oxidized protein.

Changes associated with ischemic brain disease have been proposed to be the result of alterations in calcium disposition, increase in excitoxic neurotransmitter release, production of free radicals and the attendant acidosis that results in an increase in the loosely related metals in the cell that are catalytic for the generation of oxygen free radicals. These changes are largely limited to neuronal elements. Reactive glia have been demonstrated, however, they are mostly associated with postneuronal damage.

The treatment of age related dementias have been largely limited by the inability to develop an appropriate model for the study of this condition. This is due to the fact that aging is a very complicated condition which is difficult to model, especially with the lack of specific information associated with the functional and biochemical basis of human age related dementias. The use of animal models has largely depended upon model systems used in brain studies, where the brains are not truly senescent, or the use of senescent animals, with little understanding of the origin of the senescence or, in some cases, the inability to demonstrate truly functional senescence.

The demonstration in a variety of systems, both neural and nonneural, that there is an age related enhancement of the level of oxidized protein in tissue gives rise to the possibility that age related dysfunctions in the central nervous system may be associated with the build-up of oxidized proteins and oxidized macromolecules within neurons throughout the central nervous system. The hypothesis is that cells which have a buildup of oxidized protein are less functional and less able to maintain the specified role of those cells in that particular area of the central nervous system. While this hypothesis has been suggested by several investigators, there are no reports of substantial investigations in which alterations in the oxidized protein burden of the central nervous system was manipulated and correlated with a functional outcome on the part of the animal. Such an approach, if truly associated with brain dysfunction, would provide a basis for reversing the age related neuronal deficit of cells that are still viable. Such an approach is targeted at cells which are marginally functional but still viable.

It is therefore an object of the present invention to provide compounds for use in the manufacture of a medicament for preventing or reversing damage in vivo, especially in the CNS, spinal cord and eyes. Preferably the damage results from ischemia.

Compositions for use in the inventions containing as the active ingredient a spin-trapping reagent, preferably α - phenyl butyl nitrone (PEN), or spin-trapping derivatives thereof, in a suitable pharmaceutical carrier for administration to a patient, are disclosed for treating or preventing symptoms associated with stroke or other ischemic damage, aging or other conditions associated with oxidative tissue damage. The preferred PBN compositions have the following general formula: 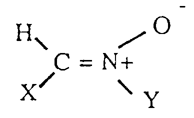
wherein:
X is phenyl or 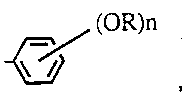
wherein R is -H, 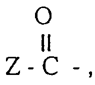
or -Z; or 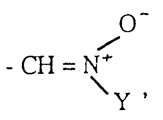
and n is a whole integer from 1 to 5; or 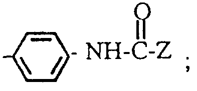
Y is a tert -butyl group that can be hydroxylated or acetylated on one or more positions; phenyl; or 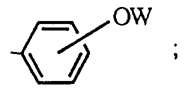
wherein W is 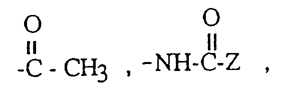 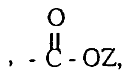
or -Z; and
Z is a C₁ to C₅ straight or branched alkyl group.

Other spin-trapping agents can also be used, such as 5,5-dimethyl pyrroline N-oxide (DMP) or α-(4-pyridyl 1-oxide)-N-tert-butylnitrone (POBN), and other spin-trapping derivatives thereof.

For the prevention or treatment of damage as a result of ischemia, the compositions are administered prior to or during ischemia in an effective dosage to prevent or reverse predisposition of the cells to damage resulting from depletion of ATP (as demonstrated by in vivo NMR) and damage from free radical generation following reperfusion. Based on animal studies, the dosage for treating damage due to stroke is in the range of 10 to 300 mg/kg. The preferred method of administration is intravenously in humans for treatment of stroke. The preferred method of administration for treating inflammation is by directed delivery to the site of inflammation.

Examples of diseases which can be treated include stroke, meningitis, progressive neuronal loss due to Parkinson's disease, senile dementia, and drug abuse, disorders arising from exposure to high pressure oxygen or enriched oxygen environments, and bleeding into nervous tissue as a result of trauma.

For treatment of aging, the compositions are preferably administered one to two times daily by oral administration, at a dosage equivalent to between one and ten milligrams PBN/70 kg of human body weight. Studies in animals demonstrate that administration of compound for a two week period reduces the level of oxidized brain enzymes to normal and restores memory to the same level as tested in young control animals. A significant reduction in oxidized proteins and memory recovery is observed as early as seven days after initiation of treatment; levels are still comparable to young controls one to three days following cessation of treatment, and partially reduced at seven days following cessation of treatment.

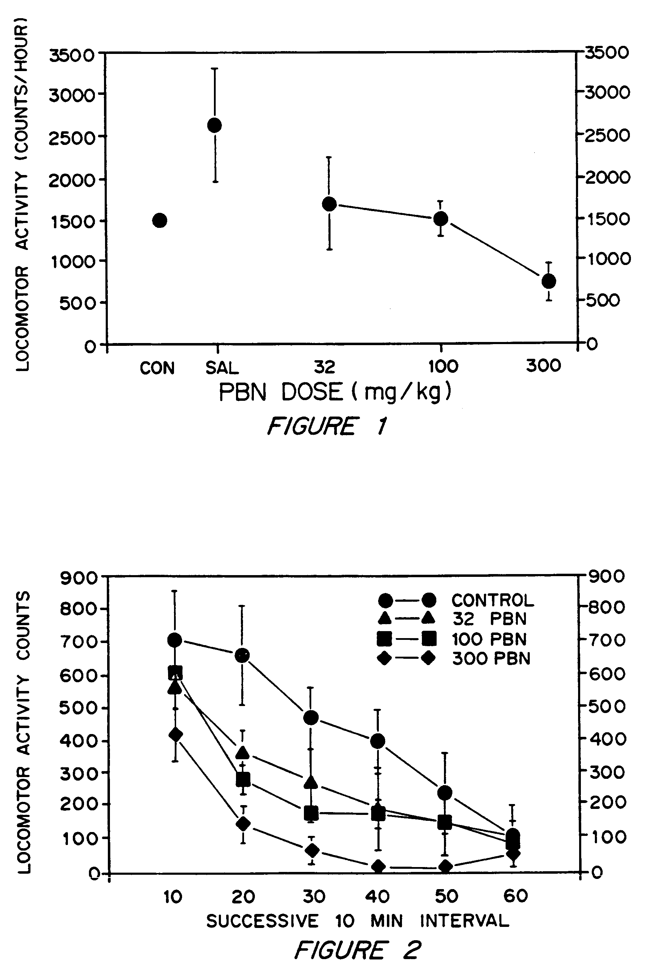

Figure 1 is a graph of the dose-effect curve for the beneficial effects of PBN on transient ischemia-induced hyperactivity, plotting locomotor activity versus PBN dose in mg/kg. Young adult gerbils were given a single dose of PBN one hr before 5 min of bilateral carotid occlusion. Twenty-four hours later the change in spontaneous locomotor activity was determined. Control (con) values represent the level of exploratory activity of a group of normal naive gerbils. Saline (Sal) values were determined in gerbils injected with saline 1 hr. prior to ischemia. Data represent the mean (± S.E.) for 6 gerbils per treatment group.

Figure 2 is a graph of the effect of PBN on the levels of activity in successive 10 min intervals for the test described in figure 1. Data represent the means (± S.E.) for 6 gerbils per treatment group: control (-●-●-); 32 mg PBN/kg (-▲-▲-); 100 mg PBN/kg (■-■); and 300 mg PBN/kg (-♢-♢-).

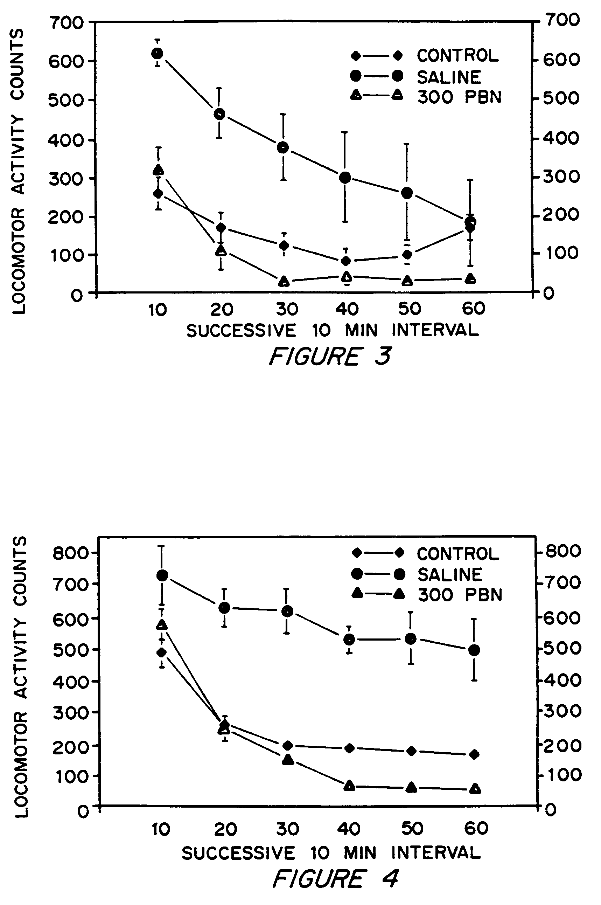

Figure 3 is a graph of the effects of PBN on the post ischemic locomotor activity of aged gerbils (greater than 20 months of age) tested 24 h after a 2 min period of ischemia, as described in figure 1. Control (--◇--◇--); saline (--0-0--); and 300 mg PBN/kg (--△--△--).

Figure 4 is a graph of the effects of administration of 300 mg PBN on 15 min ischemia-induced changes in spontaneous locomotor activity of gerbils, plotting locomotor activity counts versus time (minutes). Saline treated group (--0-0--) represents 5 of the 10 control gerbils (--◇--◇--) tested. The data for PBN (--△--△-) represents all 10 gerbils tested. Control values are for 6 gerbils. All data are expressed as the mean (±SE). Where no S.E. is indicated, it is within the dimensions of the symbol.

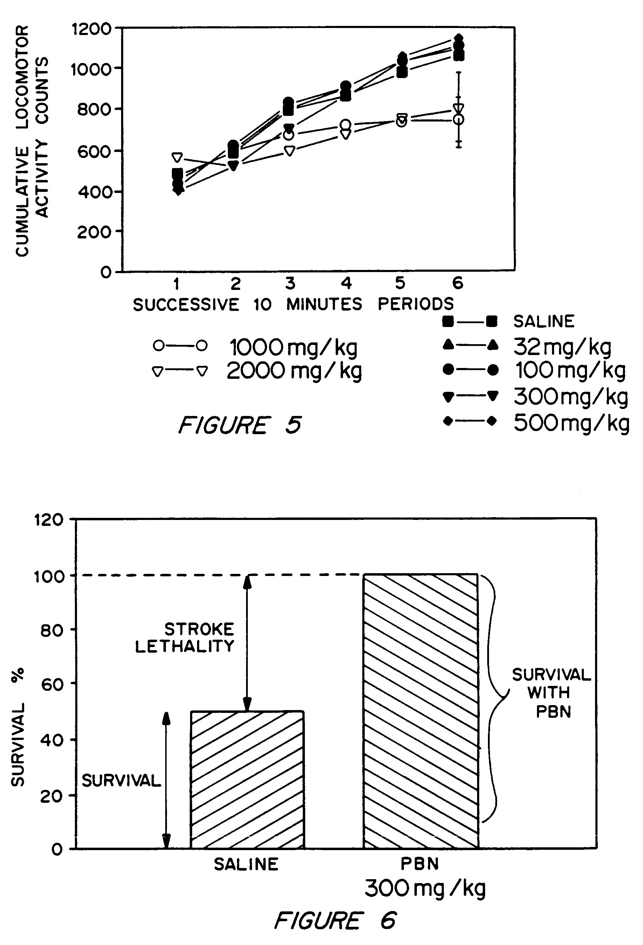

Figure 5 is a graph comparing cumulative locomotor activity counts over successive 10 minute periods for non-ischemic animals receiving saline (dark /); 32 mg PBN/kg (dark △); 100 mg PBN/kg (·); 300 mg PBN/kg (dark inverted △); 500 PBN/kg (dark ◇); 1000 mg PBN/kg (0); and 2000 mg PBN/kg (inverted △).

Figure 6 is a bar graph comparing percent survival after a lethal occlusion of the carotid arteries in gerbils treated with either saline or 300 mg PBN/kg.

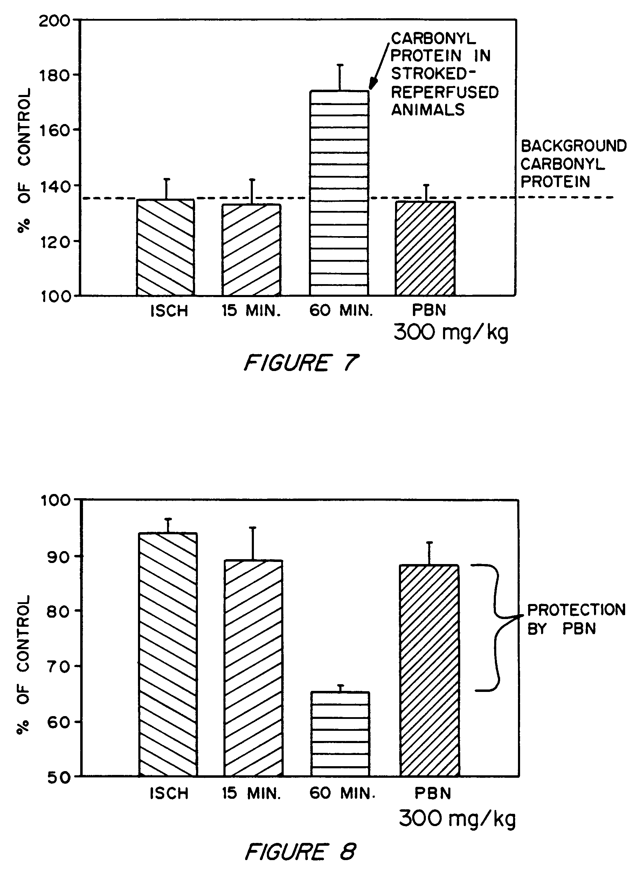

Figure 7 is a graph comparing the percent carbonyl protein of control in gerbils that have been subjected to ischemia, ischemia followed by 15 minutes reperfusion, ischemia followed by 60 minutes reperfusion, and ischemia followed by 60 minutes reperfusion with pre-ischemia administration of 300 mg PBN/kg.

Figure 8 is a graph comparing percent of glutamine synthase of control gerbils that have been subjected to ischemia, ischemia followed by 15 minutes reperfusion, ischemia followed by 60 minutes reperfusion, and ischemia followed by 60 minutes reperfusion with pre-ischemia administration of 300 mg PBN/kg.

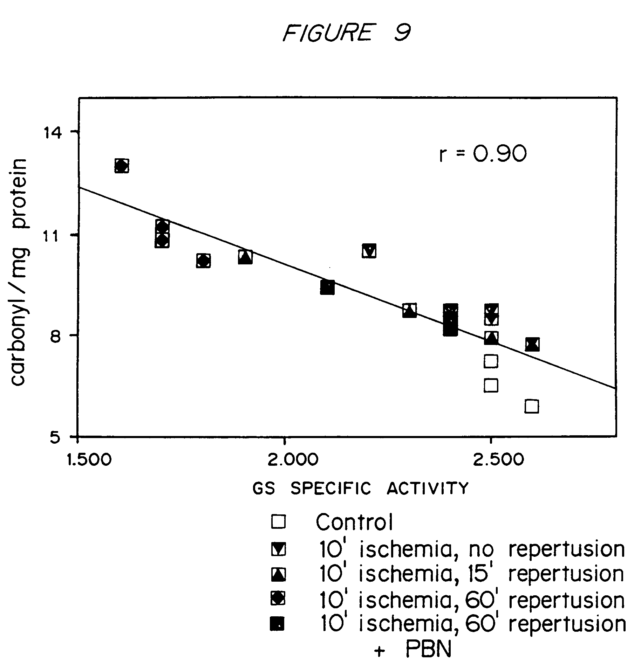

Figure 9 is a graph correlating carbonyl/ml protein versus glutamine synthetase specific activity, control (--/-/--); 10 minutes ischemia, no reperfusion (--[inverted △]-[inverted △]--), 10 minutes ischemia, 15 minutes reperfusion ([△]-[△]-), 10 minutes ischemia, 60 minutes reperfusion (--[◇]-[◇]--), and 10 minutes ischemia, 60 minutes reperfusion and PBN (dark /).

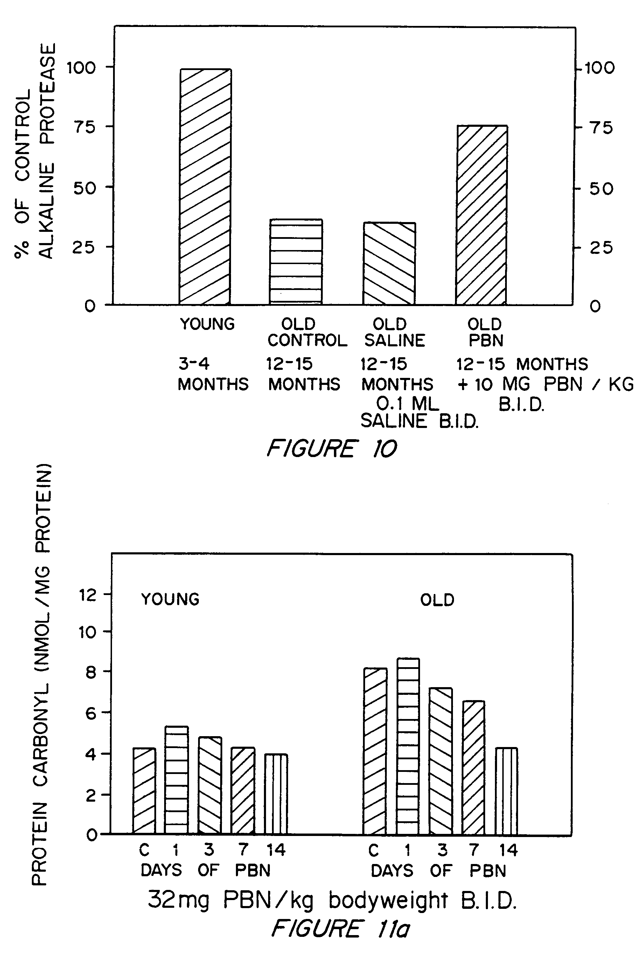

Figure 10 is a graph of the alkaline protease activity from gerbil cortex (% of young, three to four month old gerbil cortex) for young gerbils (age three to four months), old gerbils (retired breeders of twelve to fifteen months of age), old gerbils that received twice daily injections of 0.1 ml saline/kg body weight, and old gerbils that received twice daily injections of 10 mg PBN in saline/kg body weight for two weeks. Protease activity was determined using oxidized protein extracted from young gerbil cerebral cortex.

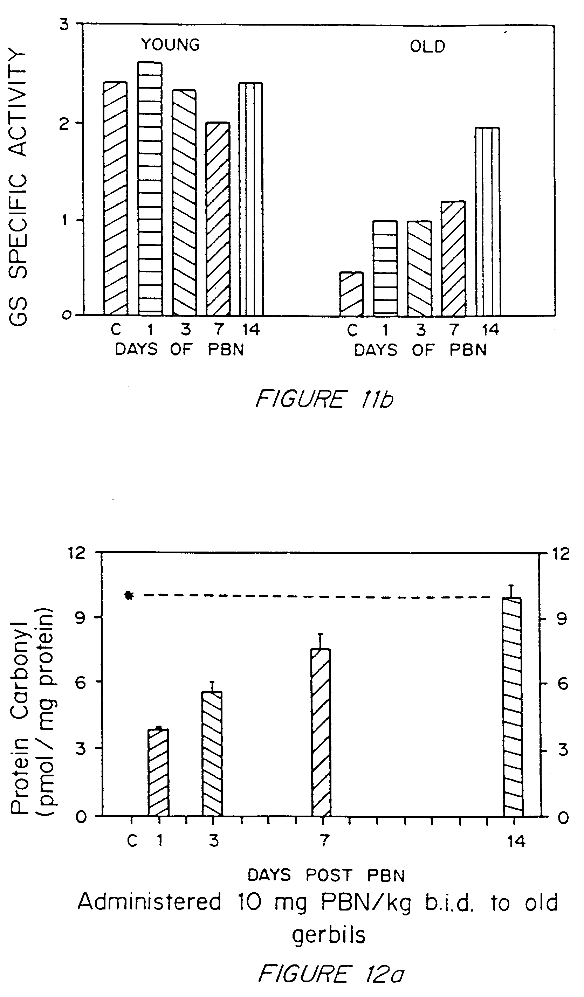

Figure 11A and Figure 11B are graphs comparing protein carbonyl activity (pmol/mg protein) (Figure 11A) and glutamine synthetase activity (Figure 11B) in the cerebral cortex (neocortex) of young adult and old gerbils over days of administration of 32 mg PBN/kg administered twice a day for one, three, seven or fourteen days. At the end of each of the days indicated, animals were decapitated and cerebral cortex removed and rapidly frozen in liquid nitrogen. Protein carbonyl content was determined using the DNPH procedure. The results demonstrate the reduction in oxidative damage to proteins and the loss of enzyme activity in gerbil cerebral cortex as a result of twice daily administration of 32 mg PBN/kg (i.p.). Each histogram is the mean of three subjects.

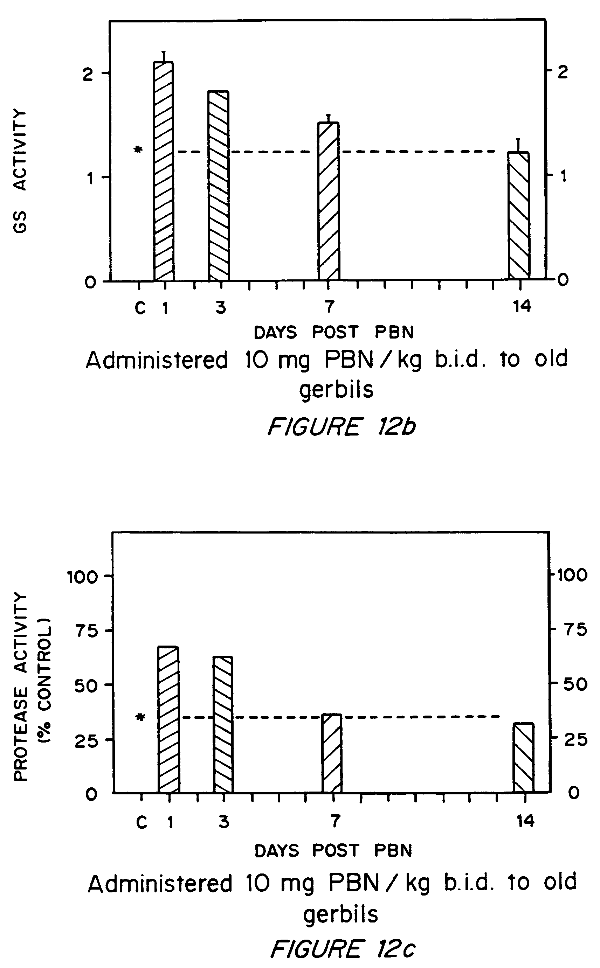

Figure 12A, 12B and 12C are graphs comparing changes in protein carbonyl (pmol/mg protein) (Figure 12A), glutamine synthetase (Figure 12B), and protease activity (% control) (Figure 12C) over days following termination of twice daily dosing with 10 mg PBN/kg body weight. Figure 12A represents the level of carbonyl in the soluble protein obtained from gerbils treated for fourteen days and tested at 1, 3, 7 and 14 days post dosing. Figure 12B is the time related decrease in cortical glutamine synthetase (GS) activity after termination of twice daily injections of PBN. Figure 12C demonstrates the time related decrease in alkaline protease activity following termination of twice daily injections of PBN. Each histogram is the mean ± standard error (S.E.) of three subjects at each of the indicated times. The asterisk and dashed line indicates the old gerbil, untreated control values for each of the measures.

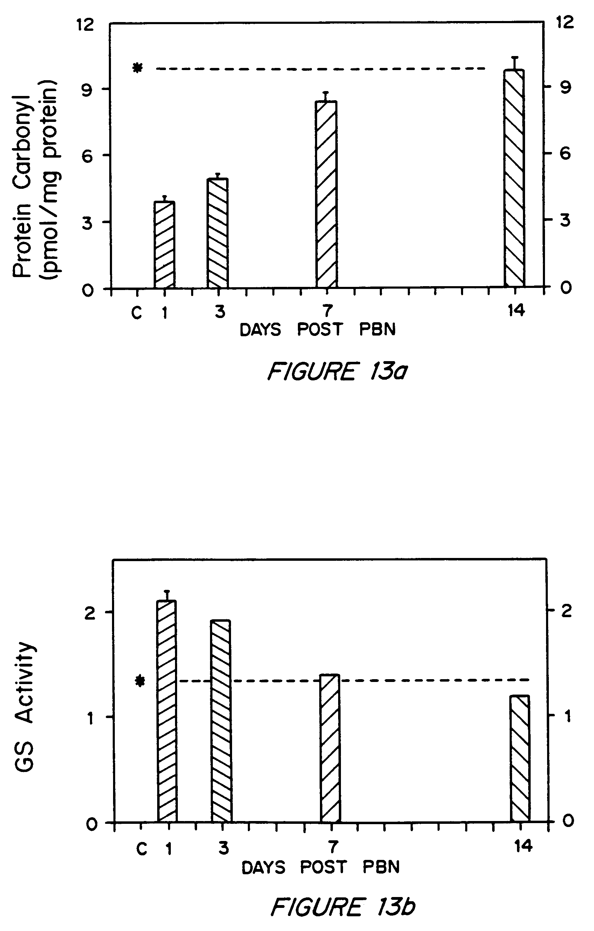

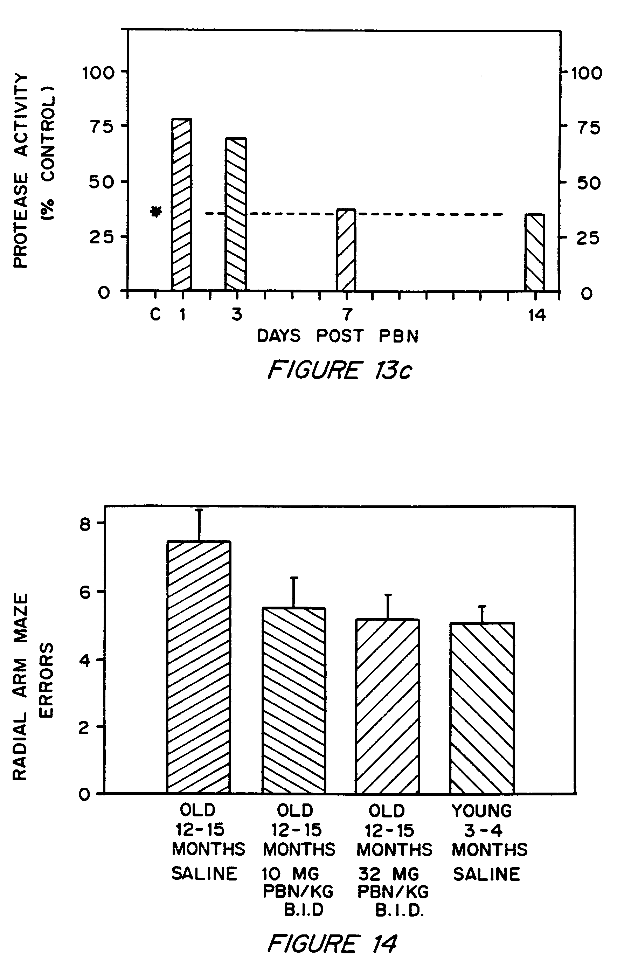

Figure 13A, 13B and 13C are graphs comparing changes in protein carbonyl (pmol/mg protein) (Figure 13A), glutamine synthetase (Figure 13B), and protease activity (% control) (Figure 13C) over days following termination of twice daily dosing with 32 mg PBN/kg body weight. Figure 13A represents the level of carbonyl in the soluble protein obtained from gerbils treated for fourteen days and tested at 1, 3, 7 and 14 days post dosing. Figure 13B is the time related decrease in cortical glutamine synthetase (GS) activity after termination of twice daily injections of PBN. Figure 13C demonstrates the time related decrease in alkaline protease activity following termination of twice daily injections of PBN. Each histogram is the mean ± S.E. of three subjects at each of the indicated times. The asterisk and dashed line indicates the old gerbil, untreated control values for each of the measures.

Figure 14 is a graph of the eight arm radial arm maze performance of young or old gerbils treated with either saline or PBN. Gerbils were placed into the central compartment of the maze with the barrier in place to limit exploration. After the barrier was removed, the number of arms re-entered and the total elapsed time before all eight arms were entered was recorded. Each histogram represents the mean ± S.E. of 18 gerbils. The animals were administered PBN twice daily (either 10 or 32 mg PBN/kg body weight) for seven days and tested at the end of seven days of dosing.

As used herein, a free radical scavenger or spin-trap reagent is a molecule that will form a stable complex with a free radical. A free radical carbon trap is a molecule in which the free radical is localized on a carbon atom. As a result of this chemical bond formation, the free radical is no longer damaging to the cell.

### Useful Spin-trapping compounds.

### PBN and derivatives thereof.

The preferred spin-trapping compounds are α-phenyl t-butyl nitrone (PBN), and derivatives thereof. PBN has no measurable effect on normal or uninjured cells. PBN is the preferred compound at this time, although a number of derivatives are also useful, including hydroxy derivatives, especially 2-, 3- or 4-hydroxy PBN and mono-, di- and trihydroxy tert-butyl nitrone; esters, especially esters which release 2-, 3, or 4-hydroxyphenyl t-butyl nitrone such as the acetoxy derivative, 2-, 3-, or 4-carboxyphenyl t-butyl nitrone, such as the ethyl derivative, or phenyl hydroxybutyl nitrone, such as the acetoxy derivative; alkoxyl derivatives, especially alkoxyl derivatives which release 2-, or 4-hydroxyphenyl t-butyl nitrone, such as the methyl derivative; and acetamide derivatives, especially acetamide derivatives which release 2-, or 4-aminophenyl t-butyl nitrone, such as the acetyl derivative; diphenyl nitrone (DPN) and the analogous diphenyl nitrone derivatives. As used herein, "PBN" refers to both phenyl t-butyl nitrone and derivatives thereof, unless otherwise stated.

The general formula for PBN and useful derivatives thereof is: 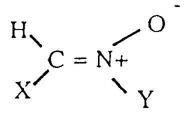
wherein:
X is phenyl or 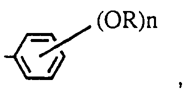
wherein R is -H, 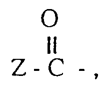
or -Z; or 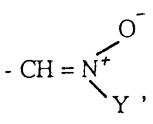
and n is a whole integer from 1 to 5; or 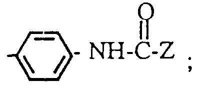
Y is a tert -butyl group that can be hydroxylated or acetylated on one or more positions; phenyl; or 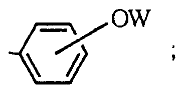
wherein W 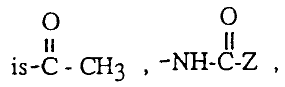 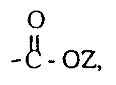
or -Z; and
Z is a C₁ to C₅ straight or branched alkyl group.

### Other spin-trapping reagents.

Other spin-trapping agents can also be used, such as 5,5-dimethyl pyrroline N-oxide (DMP) or α-(4-pyridyl 1-oxide)-N-tert-butylnitrone (POBN), and spin-trapping derivatives thereof. Derivatives are made using standard techniques, for example, for substitution of the methyl groups. The general formula for DMP is: 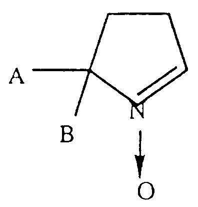
wherein A and B are independently -CH₃, -CH₂OH, -CH₂OW, or 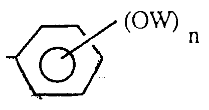
n is an integer from 1 to 5
wherein W is 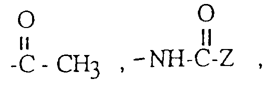 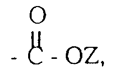
or -Z; and
Z is a C₁ to C₅ straight or branched alkyl group.

The general formula for POBN is: 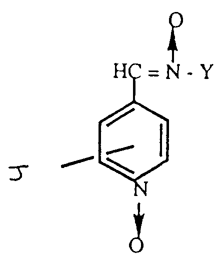
wherein
Y is a tert -butyl group that can be hydroxylated or acetylated on one or more positions; phenyl; or 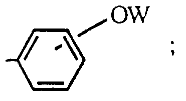
wherein W is 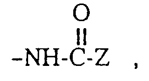 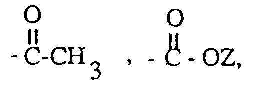
or -Z; and
J is -H,-(OR)ₙ, wherein R is -H, 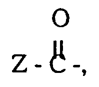
-Z, or 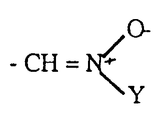
n is a whole number from 1 to 4, or 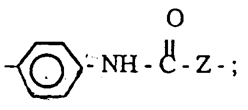
Z is a C₁ to C₅ straight branched alkyl group.

### Treatment or prevention of ischemic damage.

Following ischemia there is a significant increase in free radical production (both oxygen and carbon centered radicals). The initial damage to brain cells is thought to result from the peroxidation products with subsequent damage from secondary (carbon-centered) radicals. Eventually the metabolic and synthetic pathways are damaged to such an extent that the cell dies. PBN and functionally equivalent spin trapping derivatives that pass through the blood brain barrier, prevent cell damage during and subsequent to ischemia by decreasing or preventing ATP depletion, and still exhibit spin trapping of oxygen radicals are of benefit by bonding to the carbon-centered-free radicals to prevent further damage by the modified enzyme or other constituent.

These compounds should also be extremely useful for the prophylactic treatment of chronic or periodic cytotoxic conditions that involve free radicals. The CNS disease conditions that are expected to be treatable using such compounds include stroke, transient ischemic attacks, Alzheimer necrosis, continued cell loss in Parkinsonism, meningitis, cardiac resuscitation-induced brain damage, and damage arising as a result of trauma, especially head and spinal injuries with associated bleeding from surrounding tissues into the nervous tissues. It is expected that these compositions can also be used to treat a variety of other disorders including damage resulting from exposure to high pressure oxygen or oxygen - enriched environments, especially damage to the eyes resulting from placing very premature babies on high oxygen respirators, drug and/or alcohol abuse - induced damage to the CNS, as well as other organs, and damage resulting from exposure to ionizing radiation or photooxidation.

### Effective dosages and methods of administration of the spin-trapping agent.

α-phenyl t-butyl nitrone (PBN), and derivatives thereof, in a pharmaceutical vehicle suitable for intravenous administration are useful in preventing or reversing CNS damage following ischemia or inflammation. As used herein, ischemia is defined as a blockage of blood flow that results in a lesion in the central nervous system (CNS), including the spinal column and eyes. PBN has a number of advantages in the treatment of ischemia induced damage, including being able to pass through the blood brain barrier and having no measurable effect on normal or uninjured cells. The compositions can also contain other active agents, such as tissue plasminogen activator, streptokinase, or other clot dissolving compounds, other compounds which are oxygen radical scavengers such as mannitol or compounds which prevent peroxidase generation, such as the lazeroides.

Examples demonstrate the utility of the compositions in preventing brain damage and death in animals following blockage of blood flow through the carotid artery to the brain. Exemplary dosages of PBN ranged from 32 to 300 mg/kg of body weight in gerbils. The effective range of PBN in humans and other mammals is between approximately 10 and 300 mg/kg. The compositions can be effectively administered prior to, during or after ischemia, and prevent or decrease the extent of cellular damage.

Since the trapping of endogenous free radicals is specific for only those cells that have been exposed to the conditions that result in the production of free radicals, the traps will have little or no effect on normal cells. The beneficial effects will occur only in injured cells. Moreover, because the beneficial effect does not require the presence of specific receptors or specific enzymes, the spin traps offer a significant improvement in the treatment of ischemia-induced cellular dysfunction and necrosis.

The PBN is preferably administered systemically, most preferably intravenously, or orally, since this is the most rapid and efficient means for directing the active compound to the site of free radical generation. However, other methods of administration can be used, as when PBN is administered intraperitoneally in the examples, or intranasally through the pulmonary tract. The PBN can alternatively be administered locally, by intramuscular or subcutaneous injection, ointment, or delayed release implant.

Preferred pharmaceutical carriers for intravenous administration are saline or phosphate buffered saline at physiological pH. In the preferred method, PBN is delivered to the patient intravenously in a dosage in the range of 10 mg/kg/h, most preferably in the range of one to three mg/kg/h. The dosage can be determined for the specific disease condition and/or species by conducting measurements, as described in detail below. In general, an effective dosage is that amount of PBN that reduces enzyme activity loss by 25%, decreases cell death by 25%, and/or maintains 25% or more of normal function.

The present invention will be further understood with reference to the following non-limiting examples demonstrating methods for determining effectiveness of PBN administration for treatment of injury arising from ischemia or inflammation and demonstration in animals of the prevention and/or reversal of damage from ischemia.

### Gerbil Stroke Model.

An accepted animal model for determining the effect of a compound on injury arising from a stroke is described by Chandler, et al., J. Pharm. Methods 14, 137-146 (1985). Briefly, Mongolian gerbils are anesthetized with pentobarbital (40 mg/kg). A ventral midline incision is made in the neck. Common carotids are exposed and separated from the vago-sympathetic nerve trunk. A loop of unwaxed dental floss (Johnson and Johnson) is placed around each carotid. The ends of the floss are each passed through one of the lumens of a double lumen catheter (Dural Plastics and Engineering, Dural, NSW, Australia). The catheter and dental floss are passed through the dorsal musculature and exited at the dorsal surface of the neck. The catheter is fixed in position, directly above the carotid artery, using cyanoacrylate adhesive at the exit site. The dental floss length is marked in order to assure that the animal does not occlude the carotid during daily cleaning and exploratory activity. The ventral incision is closed with 9 mm wound clips. After 48 h following instrumentation, ischemia is produced by gently pulling the looped dental floss until the artery is occluded. Occlusion of the artery is associated with loss of consciousness, ptosis and a change in breathing pattern. Previous studies, using direct observation, have demonstrated that complete interruption of flow occurs under these conditions. The occlusion is maintained for 10 minutes and then the dental floss removed to allow complete reperfusion. After reperfusion, the catheter is trimmed flush with the surface of the neck.

The animals, male gerbils (50-60 g), are purchased from Tumblebrook Farm, West Brookfield, MA, and housed in groups of three for at least one week prior to instrumentation. Following surgery, the gerbils are singly housed in order to avoid the possible accidental induction of ischemia by cage mates. Food and water are available ad libitum in the home cage. All gerbils are maintained under a 12 h light/dark cycle.

### Example 1: Determination of the extent of peroxidation damage in brain tissue.

### Measurement of Tissue Damage by Free Radical Generation.

Tissue damage can be measured as a function of the generation of free radicals, protein carbonyl concentration increase, and decrease in glutamine synthetase enzymatic activity.

Free radicals are found in ischemia/reperfusion injured gerbil brains. There are several methods effective for measuring the generation of free radicals, including spin trapping and salicylate hydroxylation. Salicylate hydroxylation can be used to monitor hydroxyl free radicals in vivo, since hydroxylation products (2,5- and 2,3-dihydroxybenzoic acids, DHBA) are present in the brains of gerbils pretreated with salicylate. The amount of DHBA correlates closely with the amount of brain injury observed in this model, as shown by Cao, et al., Neuroscience Lett. 88, 233-238 (1988). Oxidative damage to gerbil brain proteins can be assessed by protein carbonyl group increase, which occurs as the ischemic-lesioned gerbil brain is allowed to reperfuse. Overall tissue necrosis is also indicated by a decrease in the enzymatic activity of glutamine synthetase (GS) in ischemic-lesioned gerbil brain. This decrease increases as reperfusion time increases, such that at 60 min the GS activity is decreased about 35% from normal values. The enzymatic activity of GS is highly sensitive to metal catalyzed oxidative damage. Therefore its loss supports the notion that metal catalyzed oxidative damage does occur in the reperfusion phase of the ischemic-lesioned gerbil brain. GS enzymatically converts glutamate to glutamine and thus a decrease in its activity may allow the buildup of glutamate, an excitatory toxic amino acid.

Utilizing the ischemia/reperfusion lesioned brain of gerbils, it was demonstrated that oxidative damage was occurring, with the evidence as follows: 1) increased levels of salicylate hydroxylation products, implicating increased hydroxyl free radical flux in lesioned brain, 2) increased levels of protein oxidation and a loss of glutamine synthetase activity in lesioned brain, 3) free radical formed from spin-traps in lesioned brains, 4) spintrap mediated protection from brain ischemia/reperfusion injury, and 5) an increased peroxidation capacity of brain during the reperfusion phase after an ischemic insult.

### Demonstration of in vivo oxygen free radical flux by spin-trapping and HPLC with electrochemical detection.

Spin-trapping and HPLC with electrochemical detection (LCED) are used to quantitate hydroxylation products of salicylate, formed in part by hydroxyl free radical addition, and hydroxyl free radical adducts to DNA and RNA. The extreme sensitivity of the LCED methodology, on the order of 10³ to 10⁴ gain over optical methods, is a distinct advantage when attempting to ascertain the flux of oxygen free radicals in vivo, which may only reach IO⁻⁹ M under high oxidative stress conditions. It has been demonstrated in biochemical systems that OH radicals could be trapped by salicylate and quantitated using LCED.

Electron spin resonance of brain lipid extract is measured as follows. Gerbils are administered spin-trap dissolved in saline stored cold (protected from light) I.P. 1 hr prior to treatment. Gerbils are given an ischemia/reperfusion treatment, or in the case of sham-operated controls, placed in the animal holder but no occlusion, for a defined period. The gerbils are killed by decapitation. The brains are removed and placed on a cooled stage within 2 min. The cortex of brain is isolated cold and stored at 80.C for about one week until it is extracted. The cortex is homogenized at ice temperature in a 0.5% NaCl solution (5 gm/ml) and the homogenate is then extracted with 2:1 chloroform-methanol mixture (v/v) to a final dilution of 20-fold the volume of the tissue sample. The crude extract is washed thoroughly with 0.5% NaCl (5:1, v/v) and the mixture is allowed to separate into two phases by standing at 4° C for 2 h. The organic phase is recovered and bubbled with N₂ to obtain a concentration that is needed or the solvent of the organic phase is evaporated to dryness and the residue redissolved in chloroform. Finally, the samples are transferred to a Pasteur pipette (sealed at the capillary end) and bubbled with N₂ for 5 min. The pipettes are then placed in the sample cavity of an IBM Bruker ESP300 EPR spectrometer and scanned for the presence of spin adducts. The usual spectrometer settings are as follows: Microwave power 19.8 mW; modulation amplitude 0.975G, time constant 1310.72 ms; scan range 100 G; and scan time 6 min. All spectra are recorded at room temperature. The coupling constants can be directly obtained from the instrument and since the instrument is controlled by an online computer, simulated spectra of a mixture of spin-trapped free radicals can be compared with the bona fide spectrum obtained in order to make prediction regarding the trapped free radicals. An extensive data base of spin coupling constants taken in different solvents using different systems is used for comparison purposes.

Spin-traps react with free radicals to form adducts, the electron spin resonance (ESR) spectrum, which provides a mechanism to deduce the chemical identity of the free radicals trapped. The method used for in vivo spin trapping was developed by Dr. P.B. McCay in combination with Dr. Ed Janzen, as reported by Lai, et al., Arch. Biochem. Biophys. 244, 156-160 (1986). Free radicals have been spin-trapped in ischemia/reperfusion injury in rat heart by Bolli and McCay , J, Clin. Invest. 82, 476-485 (1988).

The production of free radicals during ischemia/reperfusion induced injury in the gerbil brain was tested using spin trapping. Both treated and control animals were administered the spin trap PBN (α-phenyl-t-butyl nitrone) before the experimental treatments. The data demonstrate that large free radical signals were obtained from the extracts of brains of ischemia/reperfusion treated gerbils in contrast to very little signals from sham-operated control animals. Surprisingly, the radicals are not what would be expected when a spin-trap reacts with a free radical to produce a spin adduct. A six-line spectrum is expected from the spin-adduct of a free radical of PBN. The six-line spectrum is due to the free electron interacting with the spin 1/2 nuclear magnetic moment of the proton as well as the spin of 1 from the nitrogen of the N-oxide group. In contrast, these spectra are only three lines, and are therefore due to the free electron interacting with the N-oxide nitrogen only, which means that available protons are displaced at a distance such that they are not influencing the free electron.

The most likely explanation for the results is that PBN first traps a free radical, possibly a lipid carbon free radical, after which the spin-adduct is then oxidized to a nitrone which then traps another free radical, yielding a nitroxide having properties producing the observed spectra. Another possible explanation is that PBN is metabolized to methyl nitroso propane (MNP) which then traps a t-butyl radical to form t-butyl nitroxide.

Two other spin-traps, DMPO (5,5-dimethylpyrroline-N-oxide) and POBN (α-pyridyl-l oxide-N-t-butylnitrone), both of which have different chemical structures from PBN, yielded nitroxide spectra (very similar, 3-1ines, yet with slightly different nitrogen coupling constants and different intensities) as with PBN in the ischemia/reperfusion treated gerbils. It is pertinent to note that if the chloroform/methanol extract of brains of animals which have been pretreated with PBN is allowed to stand at room temperature for 24 hours, then the 3-1ine signal decays. If, however, more PBN is then added to the extracts and it is then incubated for 24 hours, trapped radical signals are observed in the extract of ischemic/reperfusion animals, but signals are not seen in the sham-operated controls Computer simulation of the signals developing show that three free radicals are present. One has parameters equivalent to the three line nitroxide observed previously and the other two are spin-trapped carbon free radicals, possibly lipid radicals. These observations indicate that oxidative events were started in the ischemic brains but not in the sham-operated controls and these events are carried over, at least in part, in the lipid extracts.

### Demonstration of Ischemia/reperfusion induced increased peroxidation capacity of the brain.

Brain homogenate at ice temperature does not spontaneously peroxidase, yet if the temperature is raised to 37° C there is, in contrast to almost all other tissue homogenates, spontaneous peroxidation. The rate of peroxidation varies with brain region and is highly correlated with the total iron content of the brain region. Gerbil brain homogenate peroxidizes more rapidly than rat brain. The susceptibility to peroxidation of brain homogenate after it has experienced an ischemia/reperfusion insult and the effect of time of reperfusion was determined. The data were collected from 176 gerbils. At every time point sham-operated animals were used as controls. The data show that after 10 min of ischemia there is, if reperfusion is not allowed to occur, a decrease in the rate of peroxidation. If reperfusion is allowed to occur after ischemia, then the rate of peroxidation increases significantly over sham-operated control animals in a time-course fashion. The increase is most rapid in the first 60 min then slows, reaching a maximum at 7 days following ischemia, after which it decreases to values which were still higher than controls at 14 days.

Brains from gerbils which have been pretreated with pentobarbital and then given a 15 min ischemia period followed by 15 min reperfusion have no increase in the amount of spontaneous peroxidation above sham-operated controls. Thus not only does pentobarbital protect gerbils from the damage brought about by ischemia/reperfusion injury, there is no increased peroxidation of the brain homogenate from pentobarbital treated animals as compared with untreated animals. Pre-treated animals lesioned by 15 min ischemia/15 min reperfusion had spontaneous brain homogenate peroxidation about 15% above sham-operated controls, demonstrating a direct correlation between increased brain homogenate peroxidation.

### Example 2: Demonstration of the Protective effect of spin-traps in ischemia/reperfusion induced brain injury in gerbils.

The spin-trap PBN offers considerable protection to the gerbil from brain injury brought on by an ischemia/reperfusion insult and in addition helps prevent death which often accompanies the brain injury. The data demonstrating these statements are presented in Figures 1, 2, 3 and Table 1. Table 1 shows that animals given PBN I.P., dissolved in saline, at a dosage of 300 mg/kg, 60 min prior to 15 min ischemia, which is followed by 24 h reperfusion, protected the animals from death, that is, the animals lived for longer than 7 days, the time point when lethality is evaluated. Of the control animals which received saline, 50% of the young and 100% of the old gerbils had died as of the seventh day.

Gerbils, which have received a brain ischemic insult resulting in permanent injury, first exhibit a lethargic reaction then a characteristic increase in spontaneous locomotor activity above controls at 4 hours after ischemia. The lesion-induced hyperactivity continues for several days. Drugs that protect the animals from ischemia/reperfusion injury prevent the characteristic rise in spontaneous activity. PBN was effective at various dosages in protecting against ischemia/reperfusion induced hyperactivity, as demonstrated by Table 1 and Figure 1. It can be seen that saline treated animals given a 5 min ischemia insult had hyperactivity significantly higher than sham-operated controls 24 h after ischemia. However, animals which had received PBN at 32 or 100 mg/kg 1 h prior to ischemia had no elevated hyperactivity. PBN at 300 mg/kg caused a decreased activity over controls. Data comparing activity at the 24 h reperfusion time of sham-operated controls with 2 min ischemia, 5 min ischemia and 15 min ischemia lesioned animals, which had received either saline or 300 mg/kg PBN, are shown in Figures 2, 3 and 4. The data clearly demonstrate that the high dose of PBN caused a reduction in ischemia induced hyperactivity to levels of the control or lower.

**Table 1**

| **Histopathological Scores in Gerbils 7 days** af**ter 5 min of Global Cortical Ischemia.** | |
|---|---|
| Group | Score |
| Control (no ischemia) | 0.2( .2)* |
| Saline | 4.3( .5) |
| 32 mg PBN/kg | 2.5( .7) |
| 100 mg PBN/kg | 2.8( .4) |
| 300 mg PBN/kg | 1.7( .5) |

| | |
|---|---|
| *N = 6 gerbils per group. | |

In order to obtain parameters relative to acute toxicity of PBN, total cumulative activity of normal gerbils were investigated at a wide range of concentrations. The data are shown in Figure 5. Only at PBN concentrations of 1000 mg/kg and higher was there any alteration in activity noted. PBN at 2000 mg/kg was not lethal to animals. The one noted effect at high PBN concentrations is an induced lethargic response which disappears after 24 hours.

The effect of PBN administration on percent survival of animals given lethal strokes (15 min ischemia) is shown in Figure 6. 50% of the animals given 15 minutes ischemia died. With pre-ischemic treatment with PBN, 100% of the animals survived. Ischemia/reperfusion induced oxidative damage to brain proteins.

Gerbil brains which had received various treatments were analyzed for carbonyl groups on proteins, a measure of oxidative damage to protein, and glutamine synthetase activity. The results are shown in Figure 7 and 8 and compared in Figure 9.

Figure 7 shows oxidative damage to protein as measured by carbonyl content as a percentage of a control gerbil brain given a ten minute ischemia at the indicated time of reperfusion. 300 mg PBN/kg was administered one hour prior to the onset of ischemia. Carbonyl protein content was elevated above control levels in 10 min ischemia treated brains without reperfusion. Reperfusion for 15 min did not enhance carbonyl protein above the ischemia only values. After 60 min reperfusion there was a significant increase in carbonyl protein. PBN pretreatment prevented this rise.

Figure 8 shows the oxidative damage to glutamine synthetase activity as a percentage of a control gerbil brain given a ten minute ischemia at the indicated time of reperfusion. 300 mg PBN/kg was administered one hour prior to the onset of ischemia. Glutamine synthetase (GS) activity was slightly lower than control values after ischemia with no reperfusion. With increasing reperfusion time, there was a dramatic loss in GS activity such that after 60 min reperfusion, only 65% of the activity remained. PBN pretreatment of the animals prevented this loss in GS activity. Since this enzyme is considered to be a glial cell marker, this data indicates post ischemic damage also occurs in glial cells.

Figure 9 shows that there was a good correlation between the loss of GS activity versus accumulation of carbonyl protein when all of the samples analyzed are compared.

### Ischemia/reperfusion induced gene expression.

The levels of mRNA for c-fos and c-jun were examined in gerbil brain cortex after ischemia/reperfusion. The results of 10 min of ischemia followed by 60 min of reperfusion demonstrated that both c-fos and c-jun mRNA levels are elevated within 60 min after the insult. Control animals and animals subjected to the surgery showed lower levels of c-fos mRNA was markedly enhanced. Pentobarbital pretreatment, which protects the animals from ischemia/reperfusion injury, prevented the induced expression of the c-fos gene, whereas no pretreatment caused a large increase in c-fos mRNA. PBN pretreatment suppresses the level of c-fos induction in the ischemia/reperfusion injury.

Old gerbils are much more sensitive to brain ischemia/reperfusion injury than young animals, as shown in Table 2. Data are expressed as number surviving/total tested.

**Table 2**

| **Effects of PBN pretreatments on post reperfusion lethality in young adult gerbils (3-4 months of age).** | | |
|---|---|---|
| CONDITION | PBN (300 mg/kg) | Saline |
| control | 15/15 | 15/15 |
| 15 min ischemia (young gerbils) | 10/10 | 5/10* |
| 10 min ischemia (retired breeders; (18 mo.) | 4/4 | 0/4 |

| | | |
|---|---|---|
| *All surviving gerbils were subsequently tested for behavioral effects at 24 hr. post-ischemia. | | |

### Example 3: Effectiveness of spin-trapping reagents other than PBN.

The effectiveness of two other spintraps, DMPO and POBN, in preventing the acute effects of an ischemic insult to brain was tested. The results indicate that both DMPO and POBN appear to offer some protection, but DMPO was less effective than POBN which was less effective than PBN.

### Treatment of Aging

It has also been discovered that spin-trapping agents are useful in preventing or treating symptoms associated with aging, trauma, drug administration and surgery, especially of the brain. In combination with a pharmaceutical vehicle suitable for administration to a patient, preferably by oral administration, these spin-trapping compounds are useful in preventing or reversing symptoms associated with aging, for example, increased levels of oxidized proteins, decreased enzymatic activity, and spatial and short term memory. Currently, there are no effective, non-toxic treatments for aging. Effectiveness has been demonstrated in animals after as few as seven days of administration. Effectiveness continues for at least one week after administration. Values return to pre-treatment levels after two weeks.

In one embodiment, the spin-trapping agent is administered to a patient to reverse the damage occurring as a function of age. Preliminary results indicate that there is a net increase in the oxidation of proteins and the accumulation of oxidized material in the brain. The development of senile plaque is also routinely observed in aged patients.

Other disorders are those resulting from trauma, such as a blow to the head, or from drug treatment, for example, administration of anesthesia or drug abuse, or even as a result of some types of viral infections.

It has now been determined that the level of oxidized brain protein appears to be inversely related to performance in a short term memory task and directly related to risk of stroke-induced damage and behavioral change. Increased cellular oxidation may result in one or more of the following: (a) oxidative damage to cellular proteins, which could cause a change in the regulation of ion channels, (b) a change in the rate and efficiency of signal translation and membrane depolarization, (c) significant changes in energy fluxes, which could compromise selective function, (d) alteration of RNA transcription due to oxidative damage to DNA, (e) RNA translation may be affected by either oxidation of the RNA or regulatory macromolecules, or (f) the rate of protein degradation may be altered.

Any of these changes could negatively impact on the acquisition consolidation and retrieval of information, even by interference with a single step in the learning and memory process. It is possible that oxidation of cells in a particular brain region could result in acquisition deficits, whereas oxidation of a different region could result in output deficiency. Considering the number of devastating neurodegenerative diseases, including Alzheimer's disease, this treatment could potentially be a tremendous help to people with these disorders.

Examples of other disorders that can be treated with these compositions include peripheral neuropathy of diabetes, exercise induced muscle damage and pain, and enhancement of cellular response to hormonal signals.

### Treatment of neurodegenerative disorders.

Several neurodegenerative conditions are most appropriately treated by compounds that interfere with protein oxidation. Alzheimer's disease has been associated with the accumulation of abnormal oxidized proteins or the production of abnormal proteins in areas that are pathologically affected. In addition, age related enhancement in protein oxidation occurs in all cells in the aged individual. PBN and derivatives thereof have been demonstrated to be useful in the reduction in protein oxidation and in the increase in the activity of critical enzymes within the brain of aged animals. Since this is a fundamental change in oxidative state, it is likely that PBN and other related compounds would be useful when given chronically to individuals who are in the early phases, or possibly in the late phases, of Alzheimer's disease. In addition, multi- infarct dementias should be treatable with these compounds, since they also deal with ischemia reperfusion oxidation issues.

Senile dementia has not been directly evaluated for ischemia reperfusion etiology or protein oxidation, however, it is likely that senile dementia would also be treatable with these compounds. This is based on the hypothesis that advanced age is associated with increased production of oxidized protein. In progeria, a unique condition in which aging is accelerated, Stadtman and colleagues at the NIH have demonstrated that there is a marked increase in the base-level of oxidized protein even in young adult subjects with progeria, as reported by Oliver, et al., J. Biol. Chem. 262, 5488-5491 (1987) and Starke-Reed and Oliver, Arch. Biochem. Biophys. 275, 559-567 (1989). While this is a rare condition it should also be treatable with these compositions.

Another condition which is likely to be associated with oxidative damage arising from microcirculatory difficulties is the diabetic peripheral neuropathies and vascular change. These are tragic conditions in which amputation is eventually necessary in order to save the patient. While these compounds are not likely to improve vascular flow they are likely to reduce the impact of transient changes in vascular flow which result in oxidation and damage to the peripheral nerves and also in damage to the skeletal muscle which is often associated with the condition called exercise induced or intermittent claudication. If the retinopathy associated with diabetes is also an ischemia reperfusion microcirculation problem, then the spin-trapping compounds will be useful in treating the retinal damage which occurs very frequently in diabetic patients.

### Pre-surgical preparation.

Since the status of the cell and its survival in a hypoxic or anoxic environment is dependent upon the ability of the cell to compartmentalize metals and handle oxygen in a useful manner, in contrast to peroxidation, it is expected that these compounds will be useful as presurgical preparatory medication to reduce the carbonyl load and improve the enzyme status of the patient prior to elective surgery. These compounds would also help the cells of the body achieve a higher level of enzymatic function, shorten the recuperative phase, and reduce the likelihood of any interoperative complications associated with changes in microcirculation.

### Treatment of viral infections and inflammatory disorders.

Retroviruses selectively infect certain types of cells, such as lymphocytes. An example of a retrovirus that has been the subject of much research activity is the human immunodeficiency virus (HIV), which causes Acquired Immunodeficiency Syndrome (AIDS). No means for prevention of infection has been found, although there have been numerous attempts to find a treatment. Since activation of lymphocytes is associated with the oxidation of protein and activation of lymphocytes is required prior to release of newly formed viruses, it is expected that administration of these compositions will inhibit infection and replication of lymphocytes by the viruses. The activation of the T-4 lymphocyte is associated with a cascade of biochemical intracellular changes, one of which is the production of oxidized protein. If PBN related compounds can block protein oxidation in the abnormal process then it is possible that PBN or other spin-trappin compounds could in fact interfere with the process of viral replication and/or dissemination of the virus from the host cell (T-4 lymphocyte), thereby acting as a virustatic agent by preventing the T-4 lymphocyte from releasing the newly formed viruses. This would be analogous to the use of isoniazid (INH) in the treatment of tuberculosis. At low doses INH is a tubercula static in that it reduced the infectivity and spread of the tuberculosis, thereby effectively protecting the patient from pulmonary damage.

It is important to note that the effects of the spin-trapping compounds occur in animals that have a base-level of carbonyl formation which appears to be necessary for post translational processes. Old animals have a significantly elevated level of carbonyl protein which is associated with decreased enzymatic function relative to young control animals. When young control animals are given the exact same dosage regimen, there is no significant change in enzyme activity nor is there significant change in protein carbonyl. Thus the PBN and related spin-trapping compounds are not likely to interfere with fundamental processes that are necessary for the normal cellular function.

In conclusion, a number of clinical conditions appear to have as their fundamental cause oxidation of cellular protein and enzymatic damage. Spin-trapping compounds are effective in animal models in reducing the protein oxidation and improving enzymatic function. This occurs in preparations in which the abnormal oxidized protein is modified and protected but the normal post translational oxidation is allowed to occur. This would suggest that PBN does not interfere with the normal necessary oxidation of proteins following synthesis.

### Effective dosages of Spin-trapping agent and methods of administration.

Exemplary dosages of PBN range from 0.1 to 10 mg/kg of body weight in animals. The effective dosage of PBN in humans is expected to be between approximately 1 and 10 mg/ 70 kg body weight. Toxicity tests have demonstrated that the compound is completely innocuous, with such low toxicity that it was not possible to determine an LD₅₀.

In the preferred application, the PBN or other spin-trapping agent is administered to a patient suffering from memory loss or other symptoms frequently associated with aging. Optimum results are generally observed after two weeks of daily or twice daily oral administration. The compositions can also be effectively administered prior to, during or shortly after surgery, and prevent or decrease the extent of cellular damage resulting from either the trauma or anesthesia.

Since the trapping of endogenous free radicals is specific for only those cells that have been exposed to the conditions that result in the production of free radicals, the traps have little or no effect on normal cells. The beneficial effects occur only in injured cells, and do not require the presence of specific receptors, specific enzymes, and/or specific cell types.

The PBN or other spin-trapping agent is preferably administered systemically, most preferably orally, since this is the most rapid and efficient means for delivering the active compound to the site of free radical generation. The PBN may be administered at once, or may be divided into a number of smaller doses to be administered at varying intervals of time. Other methods of administration can also be used, including subcutaneous, intravenous, and intraperitoneal administration. The concentration of active compound in the drug composition will depend on absorption, inactivation, and excretion rates of the drug as well as other factors known to those skilled in the art. The effective dosage may also be determined based on that amount required to prevent or reverse predisposition of the cells to damage resulting from depletion of ATP (as demonstrated by *in vivo* NMR) and damage from free radical generation. It is to be noted that dosage values will also vary with the condition of the patient being treated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition.

A preferred mode of administration of the active compound is in a form for oral delivery. Oral compositions will generally include an inert diluent or an edible carrier. Preferred pharmaceutical carriers for intravenous administration are saline or phosphate buffered saline at physiological pH. Since PBN degrades at pH less than approximately 3 to 4, it is preferable to administer the PBN at a pH of 4 or higher, or in combination with food, a buffering agent, or in an enteric coating. For oral delivery, the PBN may be enclosed in capsules, compressed into tablets, microencapsulated, entrapped in liposomes, in solution or suspension,alone or in combination with a substrate immobilizing material such as starch or poorly absorbable salts such as immodium. Pharmaceutically compatible binding agents can be included as part of the composition. The tablets or capsules may contain, for example, any of the following ingredients, or compounds of a similar nature: a binder such as microcrystalline cellulose, gum tragacanth or gelatin; an excipient such as starch or lactose, a disintegrating agent such as alginic acid, Primogel®, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier. In addition, dosage unit forms can contain various other materials which modify the physical form of the dosage unit, for example, coatings of sugar, shellac, or other enteric agents.

The present invention will be further understood with reference to the following non-limiting examples demonstrating methods for determining effectiveness of PBN administration for treatment or prevention and/or reversal of symptoms associated with aging.

### Example 4: Determination of brain enzyme levels in old versus young gerbils treated with PBN.

A correlations between the duration of ischemia and either the change in spontaneous behavior or the level of oxidized brain protein has previously been demonstrated in gerbils. Many other psychiatric and neurological conditions have been proposed to be the result of oxidation. Among these conditions, cellular aging has been associated with oxygen radicals and the accumulation of proteins. The level of oxidized protein, glutamine synthetase activity, brain protease activity and radial arm maze performance in young adult and retired breeder gerbils have now been compared and demonstrate that there is a direct relationship between the age of the subject and the level of oxidized brain protein, as measured using a protein carbonyl assay. Increased levels of protein carbonyl were associated with decreased glutamine synthetase activity and decreased alkaline protease activity. In contrast, there was no change in acid protease activity of retired breeders, compared to young adult gerbils. Consistent with the age-related increase in protein oxidation and enzyme damage, retired gerbils made significantly greater numbers of errors in a test of short-term memory, compared to young adult gerbils. These studies demonstrate that the functional deficits that occur as a result of aging may be associated with increased protein oxidation and decreased brain enzyme activities.

This system has been used to demonstrate the effectiveness of PBN in restoring young brain enzyme levels and short term memory to old animals. The results are shown in Figures 10 through 14, as follows. Young gerbils were obtained from Tumblebrook Farms, West Brookfield, Mass., weighing 50-60 grams and age three to four months. Control gerbils were given saline. Animals were killed by decapitation and their brains removed for analysis.

Figure 10A is a graph of the percent alkaline protease in young (three to four month old) gerbils, old (twelve to fifteen month old retired breeder) gerbils, old gerbils administered 0.1 ml saline twice daily (b.i.d.), and old gerbils administered 10 mg PBN/kg body weight b.i.d. for fourteen days.

The results demonstrate that PBN, is effective in restoring alkaline protease levels in old animals to those levels present in young animals. Figure 11A is a graph of the changes in protein oxidation from brains of young and senescent gerbils, plotting nmol protein carbonyl/mg protein versus days of treatment with PBN. The gerbils were given twice daily injections of 32 mg/kg PBN for fourteen days. Animals were killed at one, three, seven and fourteen days and protein carbonyl levels determined.

As can be seen in the figure, there is no change in the level of oxidized protein of young gerbils treated for up to 14 days with PBN. This indicates that the level of oxidized protein is likely to be a natural and necessary post translational effect, for example, if after synthesis of the protein, the protein is activated by a modification involving carbonyl oxidation. In contrast to the carbonyl level seen in young animals, control aged animals (15 months of age) have a marked increase of carbonyl content. This increased carbonyl content is responsive to treatment with PBN. Multiple days of treatment with PBN results in a progressive reduction in the level of protein carbonyl to the level seen in young animals.

The level of protein carbonyl reduction (oxidized protein burden of neurons in the brain) is only to the level of the normal young gerbil brain. Neither the levels in the young gerbil brain nor the levels in the senescent gerbil brain can be further reduced beyond this level. This observation supports the hypothesis that there is a necessary level of oxidation that occurs in cells in normal animals, which is required for cells to have "normal function", and that control aged animals (15 months of age) have a marked increase of carbonyl content. This increased carbonyl content is responsive to treatment with PBN. The ability of PBN to reduce the protein carbonyl load of cells also indicates that this is an active oxidation process which occurs at a regular or predictable rate and that there are mechanisms existent within the cells of the brain which can remove this oxidized protein if the process is interrupted.

Figure 11B compares the levels of glutamine synthetase (gs) in young and old animals and evaluates the effects of daily administration of PBN on the specific activity of the enzyme. This particular enzymatic marker has been selected because it is a highly sensitive protein to oxidation and because it is a metalloprotein that has bound to it metal which may participate in the generation of free radicals if the metal is dissociated with its binding site. Glutamine synthetase activity has been used by Stadman and colleagues (Oliver, et al., Proc. Natl. Acad. Scie. USA 87, 5144-5147 (July 1990)) as a marker enzyme for alterations following protein oxidation.

As shown in Figure 11B, the level of glutamine synthetase is lower in old gerbils (1.2) than in young adult gerbils (2.1). This is consistent with previous studies in which increases in the level of the carbonyl protein (oxidized protein in cells) is associated with a decrease in glutamine synthetase activity. In particular, if the glutamine synthetase enzyme is purified and the carbonyl content of that enzyme is evaluated, there is a marked increase in the level of oxidized protein in the presence of lowered glutamine synthetase activity. As also demonstrated in Figure 11B, repeated administration of PBN in young gerbils had no effect on glutamine synthetase activity, providing further evidence that the level of carbonyl is associated with normal function and chronic administration of PBN has no effect on either the level of carbonyl or on the marker enzyme activity. In contrast to the young gerbils, old gerbils given daily injections of PBN show a time related increase in glutamine synthetase activity that parallels the reduction in protein carbonyl content, indicating that the reduction in oxidized protein burden of cells is associated with a recovery of the enzymatic activity to the normal level seen in young adult gerbils.

It is important to note that there is no increase in enzymatic activity above that seen in young adult gerbils. Thus the treatment with PBN reverses the effect of aging on enzymatic activity but does not result in an activation of enzyme activity that exceeds the normal values.

### Example 5: Determination of residual effect of PBN on reduction of brain enzyme levels.

As shown in Figures 12A, 12B, and 12C, as compared with Figures 13A, 13B, and 13C, administration of 10 mg PBN/kg is as effective as administration of 32 mg PBN/kg body weight in restoring young enzyme levels. The appropriate dosages and full range of effective dosages for other species of animals can be determined using a similar methodology.

The time related changes in protein oxidation and enzyme activity following termination of twice daily dosing with either 10 mg PBN/kg (Figures 12A, B, and C) or 32 mg PBN/kg (Figures 13A, B, and C) are also shown by these figures. The results demonstrate that the effect of the PBN is unaltered one to three days after termination of treatment twice daily with the PBN, although the PBN itself has a half-life of three hours. At seven days, the enzyme levels are altered by approximately 50%. At fourteen days, the oxidized enzymes have returned to approximately their pre-treatment levels.

### Example 6: Demonstration of correlation between effect of PBN on brain enzymes and memory.

Figure 14 demonstrates that there is a functional counterpart to such treatment. Young and old gerbils were tested in a radial arm maze test for spacial and short term memory. Animals were placed in the central hub of a eight armed radial maze and given access to explore all eight arms of the radial arm maze. When the animal completed the test of exploring each of the eight arms, the animal was removed from the maze. The number of times that the animals reentered arms that had been previously entered was counted as an error. Under ideal conditions animals will enter each one of the eight arms but not reenter any of the arms. In many cases young adult gerbils (young) entered each of the arms without reentering any of the arms. The time required to enter all eight arms was also recorded but did not appear to determine the efficiency of the short term memory task. As can be seen in Figure 14, young gerbils made an average of 2.83 errors during the test session. In contrast. old gerbils made an average of 6.82 errors, which is a highly significant difference between the two groups.

Young adult gerbils exposed to a period of transient ischemia, which is also an oxidizing process, make a substantial number of errors with an average value of 15 errors in such a test session. During the post ischemic period, there is a marked build up of carbonyl protein and reduction in glutamine synthetase activity similar to that which is seen in aging.

Treatment of gerbils for seven days results in a marked alteration in the number of errors seen with old gerbils. Young gerbils given PBN for seven days were not significantly different from control gerbils whereas the old gerbils given PBN for seven days showed a marked reduction in the number of errors and returned to the range of performance seen with control young gerbils. Thus, there is a functional counterpart to the biochemical changes that are seen in that reduction in protein carbonyl and the increase in glutamine synthetase activity following chronic PBN treatment. This functional counterpart is demonstrated by the number of errors seen in a short term spacial memory task, the radial arm maze. It should be noted that the radial arm maze test does not require any food reinforcement or any other reward associated with the test. Naive animals are placed into the radial arm maze, are tested once and these differences are reliable on retest.

## Claims

1. The use of a compound selected from the group consisting of α-phenyl t-butyl nitrone (PBN); 5,5-dimethyl pyrroline N-oxide (DMPO), α-(4-pyridyl 1-oxide)-N-tert-butylnitrone (POBN), and derivatives thereof having spin trapping activity; as the active agent in the manufacture of a composition for *in vivo* treatment of central nervous system tissue damage.

2. The use of a compound of the general formula: 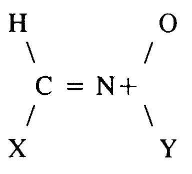 wherein:
X is phenyl or 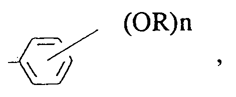 wherein R is -H, 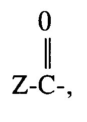 or -Z; or 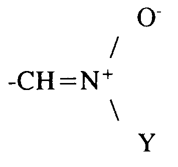 and n is a whole integer from 1 to 5; or 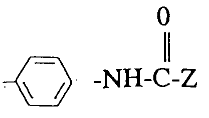 Y is a tert-butyl group that can be hydroxylated or acetylated on one or more positions; phenyl; or 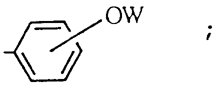 wherein W is 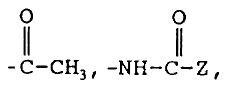 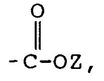 or -Z; and
Z is a C₁ to C₅ straight or branched alkyl group, as the active agent in the manufacture of a composition for in vivo treatment of central nervous system tissue damage.

3. The use according to Claim 1 or 2 wherein the central nervous tissue damage is damage resulting from ischemia.

4. The use of Claim 1, 2 or 3 wherein the central nervous system tissue is spinal cord.

5. The use of Claim 1, 2 or 3 wherein the central nervous system is tissue of the eye.

6. The use according to Claim 2 wherein the phenyl butyl nitrone derivatives are selected from hydroxy PBNs, PBN esters, acetoxy PBNs, alkyl PBNs, alkoxyl PBNs and phenyl PBNs.

7. The use according to Claim 2 wherein the PBN derivative is functionalized to release *in vivo* a compound selected from 2-, 3-, and 4-hydroxyphenyl t-butyl nitrone; 2-, 3-, and 4-carboxyphenyl t-butyl nitrone; and 2-, 3-, and 4-aminophenyl t-butyl nitrone.

8. The use according to Claim 2 wherein the active agent is α-phenyl t-butyl nitrone.

9. The use of a compound of has the general formula: 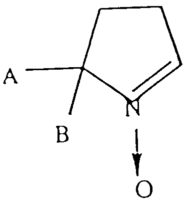 wherein A and B are independently -CH₃, -CH₂OH, -CH₂OW, or 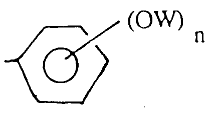 n is an integer from 1 to 5
wherein W is 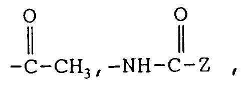 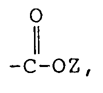 or -Z; and
Z is a C₁ to C₅ straight or branched alkyl group, as the active agent in the manufacture of a composition for in vivo treatment of central nervous system tissue damage.

10. The use according to claim 9 wherein the active agent is 5,5-dimethyl pyrroline N-oxide.

11. The use according to Claim 1 wherein the active agent has the general formula: 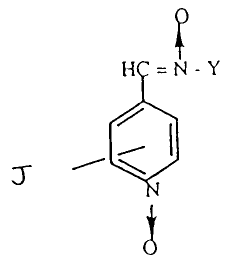 wherein
Y is a tert-butyl group that can be hydroxylated or acetylated on one or more positions; phenyl; or 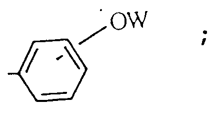 wherein W is 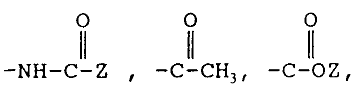 or-Z; and J is H, -(OR)ₙ,
wherein R is -H, 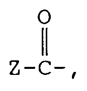 -Z, or 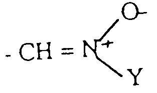 n is a whole number from 1 to 4, or 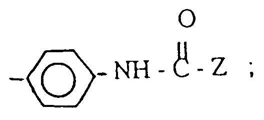 Z is a C₁ to C₅ straight or branched alkyl group.

12. The use according to Claim 11 wherein the active agent is α-(4-pyridyl 1-oxide)-N-tert-butylnitrone.

13. The use according to any one of the preceding claims wherein the active agent is in a pharmaceutical carrier for parenteral administration to a patient, in a form delivering an effective dosage to prevent central nervous system tissue damage.

14. The use according to any one of Claims 1 to 12 wherein the active agent is in a pharmaceutical carrier for parenteral administration to a patient, in a form delivering an effective dosage to restore the brain enzyme levels of an old animal to that of a young animal.

15. The use according to any one of the preceding claims wherein the active agent is provided in combination with a pharmaceutical carrier selected from the group consisting of microspheres, liposomes, immobilizing substrates, polymeric matrices and buffering agents.

16. The use according to Claim 1 wherein the active agent is provided in dosage units suitable for the administration of 1 to 300 mg/kg body weight.

17. The use according to Claim 16 wherein the active agent is provided in dosage units suitable for the administration of 1 to 10 mg/kg body weight.

18. The use according to Claim 1 wherein the active agent is provided in dosage units suitable for the administration of 10 to 300 mg PBN/kg body weight.

19. The use according to Claim 1 wherein the composition is in a form suitable for administration during or immediately after a stroke.

20. The use according to Claim 1 wherein the central nervous system tissue damage is damage resulting from progressive neuronal disorder.

21. The use according to Claim 20 wherein the progressive neuronal disorder is Parkinson's disease.

22. The use according to Claim 20 wherein the progressive neuronal disorder is Alzheimer's disease.

23. The use according to Claim 1 wherein the central nervous system damage is damage resulting from exposure to high pressure oxygen or an oxygen enriched environment.

24. The use according to Claim 1 wherein the central nervous system damage is damage resulting from exposure to a chemical agent.

## Patentansprüche

1. Verwendung einer Verbindung, die aus der Gruppe gewählt wird, die aus α-Phenyl-tert.-butylnitron (PBN); 5,5-Dimethylpyrrolin-N-oxid (DMPO), α-(4-Pyridyl-1-oxid)-N-tert.-butylnitron (POBN) und Derivaten davon mit *Spin-trapping*-Aktivität (Spin-"Einfang"-Aktivität) besteht, als der wirksame Bestandteil bei der Herstellung einer Zusammensetzung zur *In-vivo*-Behandlung von Gewebsschädigung des Zentralnervensystems (ZNS).

2. Verwendung einer Verbindung der allgemeinen Formel: 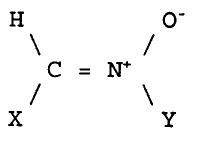 worin:
X Phenyl oder 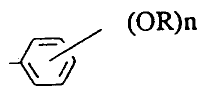 ist, worin R -H, 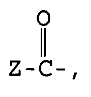 oder -Z, oder 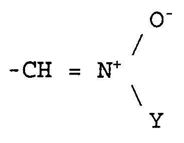 ist,
und n eine ganze Zahl von 1 bis 5 oder 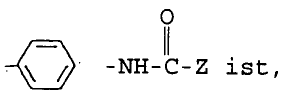 Y eine tert.-Butyl-Gruppe, die an einer oder mehreren Stellungen hydroxyliert oder acetyliert sein kann; Phenyl, oder 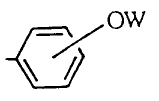 ist,
worin W 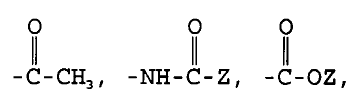 oder -Z ist; und
Z eine geradkettige oder verzweigte C₁- bis C₅-Alkylgruppe ist, als der wirksame Bestandteil bei der Herstellung einer Zusammensetzung zur *In-vivo*-Behandlung von Gewebsschädigung des ZNS.

3. Verwendung nach Anspruch 1 oder 2, worin die Gewebsschädigung des ZNS eine auf Ischämie zurückführbare Schädigung ist.

4. Verwendung von Anspruch 1, 2 oder 3, worin das ZNS-Gewebe Rückenmark ist.

5. Verwendung von Anspruch 1, 2 oder 3, worin das ZNS Gewebe des Auges ist.

6. Verwendung nach Anspruch 2, worin die Phenylbutylnitron-Derivate aus Hydroxy-PBN, PBN-Estern, Acetoxy-PBN, Alkyl-PBN, Alkoxyl-PBN und Phenyl-PBN ausgewählt sind.

7. Verwendung nach Anspruch 2, worin das PBN-Derivat zur Freisetzung *in vivo* einer aus 2-, 3- und 4-Hydroxyphenyl-tert.-butylnitron, 2-, 3- und 4-Carboxyphenyl-tert.-butylnitron und 2-, 3- und 4-Aminophenyl-tert.-butylnitron ausgewählten Verbindung funktionalisiert ist.

8. Verwendung nach Anspruch 2, worin der wirksame Bestandteil α-Phenyl-tert.-butylnitron ist.

9. Verwendung einer Verbindung der allgemeinen Formel: 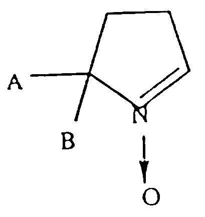 worin A und B unabhängig voneinander -CH₃, -CH₂OH, -CH₂OW oder 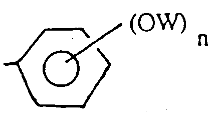 sind, n eine ganze Zahl von 1 bis 5 ist,
worin W 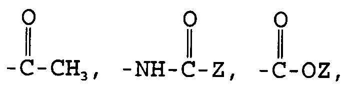 oder -Z ist; und
Z eine geradkettige oder verzweigte C₁- bis C₅-Alkylgruppe ist, als der wirksame Bestandteil bei der Herstellung einer Zusammensetzung zur *In-vivo*-Behandlung von Gewebsschädigung des ZNS.

10. Verwendung nach Anspruch 9, worin der wirksame Bestandteil 5,5-Dimethylpyrrolin-N-oxid ist.

11. Verwendung nach Anspruch 1, worin der wirksame Bestandteil die allgemeine Formel wie folgt aufweist: 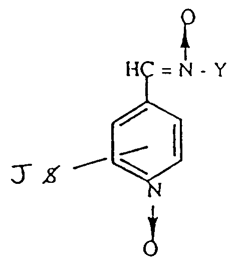 worin Y eine tert.-Butylgruppe, die an einer oder mehreren Stellungen hydroxyliert oder acetyliert sein kann,
Phenyl oder 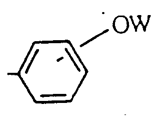 ist;
worin W 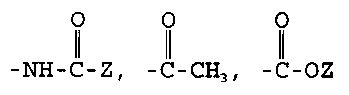 oder -Z ist, und J H,-(OR)ₙ ist, worin R -H, 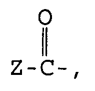 -Z, oder 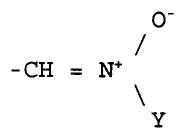 ist,
n eine ganze Zahl von 1 bis 4, oder 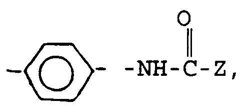 und
Z eine geradkettige oder verzweigte C₁- bis C₅-Alkylgruppe ist.

12. Verwendung nach Anspruch 11, worin der wirksame Bestandteil α-(4-Pyridyl-1-oxid)-N-tert.-butylnitron ist.

13. Verwendung nach einem der vorangegangenen Ansprüche, worin der wirksame Bestandteil in einem pharmazeutischen Träger zur parenteralen Verabreichung an einen Patienten in einer Form vorliegt, in der eine wirksame Dosierung zur Verhinderung einer ZNS-Gewebsschädigung abgegeben wird.

14. Verwendung nach einem der Ansprüche 1 bis 12, worin der wirksame Bestandteil in einem pharmazeutischen Träger zur parenteralen Verabreichung an einen Patienten in einer Form vorliegt, in der eine wirksame Dosierung zur Wiederherstellung der Enzymspiegel im Gehirn eines alten Tieres auf die eines jungen Tieres abgegeben wird.

15. Verwendung nach einem der vorangegangenen Ansprüche, worin der wirksame Bestandteil in Kombination mit einem pharmazeutischen Träger bereitgestellt wird, welcher aus der aus Mikrosphären, Liposomen, Immobilisierungssubstraten, polymeren Matrices und Puffersubstanzen bestehenden Gruppe ausgewählt wird.

16. Verwendung nach Anspruch 1, worin der wirksame Bestandteil in für die Verabreichung geeigneten Dosierungseinheiten von 1 bis 300 mg/kg Körpergewicht bereitgestellt wird.

17. Verwendung nach Anspruch 16, worin der wirksame Bestandteil in für die Verabreichung geeigneten Dosierungseinheiten von 1 bis 10 mg/kg Körpergewicht bereitgestellt wird.

18. Verwendung nach Anspruch 1, worin der wirksame Bestandteil in für die Verabreichung geeigneten Dosierungseinheiten von 10 bis 300 mg PBN/kg Körpergewicht bereitgestellt wird.

19. Verwendung nach Anspruch 1, worin die Zusammensetzung in einer Form vorliegt, die für die Verabreichung während oder sofort nach einem Schlaganfall geeignet ist.

20. Verwendung nach Anspruch 1, worin die ZNS-Gewebsschädigung eine Schädigung ist, die sich auf eine progressive neuronale Erkrankung zurückführen läßt.

21. Verwendung nach Anspruch 20, worin die progressive neuronale Erkrankung die Parkinson-Krankheit ist.

22. Verwendung nach Anspruch 20, worin die progressive neuronale Erkrankung die Alzheimer-Krankheit ist.

23. Verwendung nach Anspruch 1, worin die ZNS-Schädigung eine Schädigung aufgrund von Exposition gegenüber Hochdrucksauerstoff oder einer mit Sauerstoff angereicherten Umgebung ist.

24. Verwendung nach Anspruch 1, worin die ZNS-Schädigung eine Schädigung aufgrund von Exposition gegenüber einem chemischen Mittel ist.

## Revendications

1. L'utilisation d'un composé choisi dans le groupe consistant en α-phényl t-butyl nitrone (PBN); le 5,5-diméthyl pyrroline N-oxyde (DMPO), le α-(4-pyridyl 1-oxyde)-N-tert-butylnitrone (POBN) et leurs dérivés ayant une activité de captage de spin; en tant que principe actif dans la fabrication d'une composition pour le traitement in vivo des lésions tissulaires du système nerveux central.

2. L'utilisation d'un composé de formule générale: 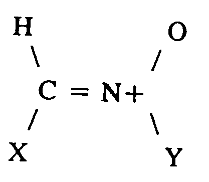 où X est du phényle ou 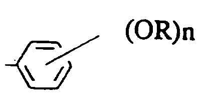 où R est -H 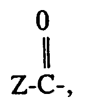 or -Z; or 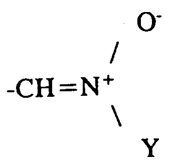 et n est un nombre entier entre 1 et 5 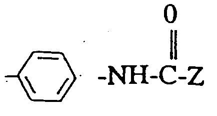 Y est un groupe tert-butyl qui peut être hydroxylé ou acétylé en une ou plusieurs positions; du phényle; ou 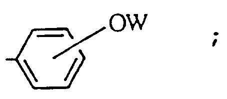 où W est 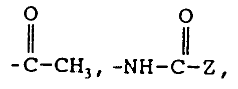 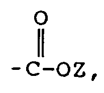 or -Z;
et
Z est un groupe alcoyle à chaîne linéaire ou ramifiée C1 à C5, en tant que principe actif utilisé dans la fabrication d'une composition pour le traitement in vivo de lésions tissulaires du système nerveux central.

3. L'utilisation selon la revendication 1 ou 2 où la lésion tissulaire du système nerveux central est une lésion résultant d'une ischémie.

4. L'utilisation selon la revendication 1, 2 ou 3 où le tissu du système nerveux central est la moelle épinière.

5. L'utilisation selon la revendication 1, 2 ou 3 où le tissu du système nerveux central est le tissu de l'oeil.

6. L'utilisation selon la revendication 2 où les dérivés de phénylbutylnitrone sont choisis dans le groupe consistant en hydroxy PBN, esters de PBN, acétoxy PBN, alcoyle PBN, alkoxyl PBN et phényle PBN.

7. L'utilisation selon la revendication 2 où le dérivé de PBN est fonctionnalisé pour libérer in vivo un composé choisi dans le groupe consistant en 2-, 3- et 4-hydroxyphényl t-butyl nitrone; le 2-, 3- et 4-carboxyphényl t-butyl nitrone et le 2-, 3- et 4-aminophényl t-butyl nitrone.

8. L'utilisation selon la revendication 2 où le principe actif est de la α-phényl t-butyl nitrone.

9. L'utilisation d'un composé de formule générale: 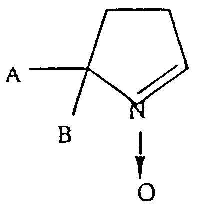 où A et B sont indépendamment -CH₃, -CH₂OH, -CH₂OW, ou 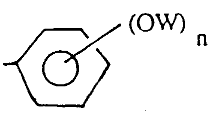 n est un nombre entier entre 1 et 5
où W est 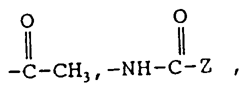 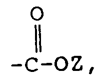 or-Z;
and
Z est un groupe alcoyle à chaîne linéaire ou ramifiée C1 à C5, en tant que principe actif utilisé dans la fabrication d'une composition pour le traitement in vivo de lésions tissulaires du système nerveux central.

10. L'utilisation selon la revendication 9 dans laquelle le principe actif est du 5,5-diméthyl pyrroline N-oxyde.

11. L'utilisation selon la revendication 1 dans laquelle le principe actif est de formule générale: 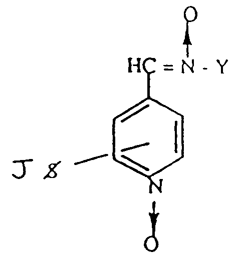 où Y est un groupe tert-butyl qui peut être hydroxylé ou acétylé en une ou plusieurs positions; du phényle; ou 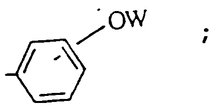 où W est 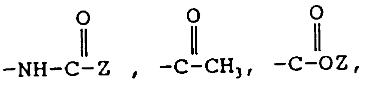 or-Z; et J est H,-(OR)ₙ, où R est -H 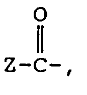 -Z, or 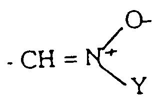 n est un nombre entier entre 1 et 4 ou 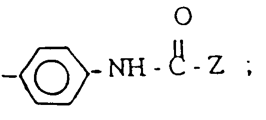 Z est un groupe alcoyle à chaîne linéaire ou ramifiée C1 à C5.

12. L'utilisation selon la revendication 1 dans laquelle le principe actif est le α-(4-pyridyl 1-oxyde)-N-tert-butylnitrone.

13. L'utilisation selon l'une quelconque des revendications précédentes dans laquelle le principe actif est contenu dans un support pharmaceutique pour l'administration parentérale à un patient sous une forme délivrant une posologie efficace pour prévenir les lésions tissulaires du système nerveux central.

14. L'utilisation selon l'une quelconque des revendications 1 à 12 dans laquelle le principe actif est contenu dans un support pharmaceutique pour l'administration parentérale à un patient sous une forme délivrant une posologie pour ramener les taux d'enzymes du cerveau chez un animal âgé aux taux d'un animal jeune.

15. L'utilisation selon l'une quelconque des revendications précédentes dans laquelle le principe actif est fourni en association avec un support pharmaceutique choisi dans le groupe consistant en microsphères, liposomes, substrats d' immobilisation, matrices polymères et agents tampons.

16. L'utilisation selon la revendication 1 dans laquelle le principe actif est fourni en doses unitaires conçues pour l'administration de 1 à 300 mg/kg de poids corporel.

17. L'utilisation selon la revendication 16 dans laquelle le principe actif est fourni en doses unitaires conçues pour l'administration de 1 à 10 mg/kg de poids corporel.

18. L'utilisation selon la revendication 1 dans laquelle le principe actif est fourni en doses unitaires conçues pour l'administration de 10 à 300 mg de PBN/kg de poids corporel.

19. L'utilisation selon la revendication 1 dans laquelle la composition est dans une forme conçue pour l'administration au cours de, ou immédiatement après un ictus.

20. L'utilisation selon la revendication 1 dans laquelle la lésion tissulaire du système nerveux central est une lésion résultant d'un trouble neuronal progressif.

21. L'utilisation selon la revendication 20 dans laquelle le trouble neuronal progressif est la maladie de Parkinson.

22. L'utilisation selon la revendication 20 dans laquelle le trouble neuronal progressif est la maladie d'Alzheimer.

23. L'utilisation selon la revendication 1 dans laquelle la lésion du système nerveux central est une lésion résultant de l'exposition à de l'oxygène à haute pression ou à un environnement enrichi en oxygène.

24. L'utilisation selon la revendication 1 dans laquelle la lésion du système nerveux central est une lésion due à l'exposition à un agent chimique.
